(19) **Europäisches Patentamt**

**European Patent Office**

**Office européen des brevets**

(11) **EP 1 347 747 B1**

(12) **EUROPEAN PATENT SPECIFICATION**

(45) Date of publication and mention
of the grant of the patent:
**08.03.2006 Bulletin 2006/10**

(51) Int Cl.:
*A61K 9/20* (2006.01)       *A61K 9/51* (2006.01)
*A61K 31/496* (2006.01)      *A61K 31/55* (2006.01)
*A61K 31/573* (2006.01)      *A61K 31/192* (2006.01)

(21) Application number: **01998077.0**

(22) Date of filing: **20.12.2001**

(86) International application number:
**PCT/US2001/049737**

(87) International publication number:
**WO 2002/055059 (18.07.2002 Gazette 2002/29)**

(54) **METHOD FOR PREPARING SUBMICRON PARTICLE SUSPENSIONS OF PHARMACEUTICAL AGENTS**

VERFAHREN ZUR HERSTELLUNG VON SUBMIKROPARTIKEL-SUSPENSIONEN PHARMAZEUTISCHER SUBSTANZEN

PREPARATION DE SUSPENSIONS DE PARTICULES SUBMICRONIQUES D'AGENTS PHARMACEUTIQUES

(84) Designated Contracting States:
**AT BE CH CY DE DK ES FI FR GB GR IE IT LI LU MC NL PT SE TR**

(30) Priority: **22.12.2000 US 258160 P**
**05.06.2001 US 874799**
**05.06.2001 US 874637**
**05.06.2001 US 874499**
**17.09.2001 US 953979**
**19.10.2001 US 35821**
**12.12.2001 US 21692**

(43) Date of publication of application:
**01.10.2003 Bulletin 2003/40**

(60) Divisional application:
**05077486.8**

(73) Proprietor: **Baxter International Inc.**
**Deerfield, Illinois 60015 (US)**

(72) Inventors:
• **KIPP, James, E.**
**Wauconda, IL 60084 (US)**

• **WONG, Joseph, Chung, Tak**
**Gurnee, IL 60031 (US)**
• **DOTY, Mark, J.**
**Grayslake, IL 60030 (US)**
• **REBBECK, Christine, L.**
**Algonquin, IL 60102 (US)**
• **BRYNJELSEN, Sean**
**Lake-In-The-Hills, Il 60156 (US)**
• **WERLING, Jane**
**Arlington Heights, IL 60005 (US)**
• **SRIRAM, Rajaram**
**Glenview, IL 60025 (US)**

(74) Representative: **Dee, Ian Mark**
**Eric Potter Clarkson LLP**
**Park View House**
**58 The Ropewalk**
**Nottingham NG1 5DD (GB)**

(56) References cited:
EP-A- 0 169 618          WO-A-98/57967
WO-A-99/16443            WO-A-99/33467
US-A- 4 606 940          US-A- 4 826 689
US-A- 5 122 543          US-A- 5 145 684
US-A- 5 780 062          US-A- 6 039 981

**Description**

**BACKGROUND OF THE INVENTION:**

Technical Field

The present invention relates to the preparation of a pharmaceutically active compound. More particularly the invention relates to the manufacture of nanosuspensions of the pharmaceutically active compound for parenteral or oral delivery.

Background Art

There is an ever increasing number of pharmaceutical drugs being formulated that are poorly soluble or insoluble in aqueous solutions. Such drugs provide challenges to delivering them in an injectable form such as through parenteral administration. Drugs that are insoluble in water can have significant benefits when formulated as a stable suspension of sub-micron particles. Accurate control of particle size is essential for safe and efficacious use of these formulations. Particles must be less than seven microns in diameter to safely pass through capillaries without causing emboli (Allen et al., 1987; Davis and Taube, 1978; Schroeder et al., 1978; Yokel et al., 1981).

One approach to delivering an insoluble drug is disclosed in United States Patent No. 2,745,785. This patent discloses a method for preparing crystals of penicillin G suitable for parenteral administration. The method includes the step of recrystallizing the penicillin G from a formamide solution by adding water to reduce the solubility of the penicillin G. The '785 Patent further provides that the penicillin G particles can be coated with wetting agents such as lecithin, or emulsifiers, surface-active and defoaming agents, or partial higher fatty acid esters of sorbitan or polyoxyalkyklene derivatives thereof, or aryl alkyl polyether alcohols or salts thereof. The '785 patent further discloses micronizing the penicillin G with an air blast under pressure to form crystals ranging from about 5 to 20 microns.

Another approach is disclosed in United States Patent No. 5,118,528 which discloses a process for preparing nanoparticles. The process includes the steps of: (1) preparing a liquid phase of a substance in a solvent or a mixture of solvents to which may be added one or more surfactants, (2) preparing a second liquid phase of a non-solvent or a mixture of non-solvents, the non-solvent is miscible with the solvent or mixture of solvents for the substance, (3) adding together the solutions of (1) and (2) with stirring; and (4) removing of unwanted solvents to produce a colloidal suspension of nanoparticles. The '528 Patent discloses that it produces particles of the substance smaller than 500 nm without the supply of energy. In particular the '528 Patent states that it is undesirable to use high energy equipment such as sonicators and homogenizers.

United States Patent No. 4,826.689 and EP-A-0,169,618 disclose a method for making uniformly sized particles from water-insoluble drugs or other organic compounds. First, a suitable solid organic compound is dissolved in an organic solvent, and the solution can be dilated with a non-solvent. Then, an aqueous precipitating liquid is infused, precipitating non-aggregated particles with substantially uniform mean diameter. The particles are then separated from the organic solvent. Depending on the organic compound and the desired particle size, the parameters of temperature, ratio of non-solvent to organic solvent, infusion rate, stir rate, and volume can be varied according to the invention. The '689 and '618 Patents disclose this process forms a drug in a metastable state which is thermodynamically unstable and which eventually converts to a more stable crystalline state. The '689 and '618 Patents discloses trapping the drug in a metastable state in which the free energy lies between that of the starting drug solution and the stable crystalline form. The '689 and '618. Patents disclose utilizing crystallization inhibitors (e.g., polyvinylpyrrolidinone) and surface-active agents (e.g., poly(oxyethylene)-co-oxypropylene)) to render the precipitate stable enough to be isolated by centrifugation, membrane filtration or reverse osmosis.

In U.S. Patent Nos. 5,091,188; 5,091,187 and 4,725,442 which disclose (a) either coating small drug particles with natural or synthetic phospholipids or (b) dissolving the drug in a suitable lipophilic carrier and forming an emulsion stabilized with natural or semisynthetic phospholipids. One of the disadvantages of these approaches is they rely on the quality of the raw material of the drug and do not disclose steps of changing the morphology of the raw material to render the material in a friable, more easily processed form.

Another approach to providing insoluble durgs for parenteral delivery is disclosed in U.S. Patent No. 5,145,684. The '684 Patent discloses the wet milling of an insoluble drug in the presence of a surface modifier to provide a drug particle having an average effective particle size of less than 400 nm. The '684 Patent emphasizes the desirability of not using any solvents in its process. The '684 Patent discloses the surface modifier is adsorbed on the surface of the drug particle in an amount sufficient to prevent agglomeration into larger particles.

Yet another attempt to provide insoluble drugs for parenteral delivery is disclosed in U.S. Patent Nos. 5,922,355. The '355 Patent discloses providing submicron sized particles of insoluble drugs using a combination of surface modifiers and a phospholipid followed by particle size reduction using techniques such as sonication, homogenization, milling, microfluidization, precipitation or recrystallization. There is no disclosure in the '355 Patent of changing process conditions to make crystals in a more friable form.

United States Patent No. 5,780,062 discloses a method of preparing small particles of insoluble drugs by (1) dissolving the drug in a water-miscible first solvent, (2) preparing a second solution of a polymer and an amphiphile in an aqueous second solvent in which the drug is substantially insoluble whereby a polymer/amphiphile complex is formed and (3) mixing the solutions from the first and second steps to precipitate an aggregate of the drug and polymer/amphiphile complex.

United States Patent No. 5,858,410 discloses a pharmaceutical nanosuspension suitable for parentreal administration The '410 patent discloses subjecting at least one solid therapentically active compound dispersed in a solvent to high pressure homogenization in a piston-gap homogenizer to form particles having an average diameter, determined by photon correlation spectroscopy (PCS) of 10 nm to 1000 rim, the proportion of particles larger than 5 $\mu$m in the total population being less than 0.1% (number distribution determined with a Coulter counter), without prior conversion into a melt, wherein the active compound is sold at room temperature and is insoluble, only sparingly soluble or moderately soluble in water, aqueons media and/or organic solvents. The Examples in the '410 Patent disclose jet milling prior to homogenization.

United States Patent No. 4,997,434 discloses a method for making uniformly sized particles from solid compounds. The method of the '454 Patent includes the steps of dissolving the solid compound in a suitable solvent followed by infusing precipitating liquid thereby precipitating non-aggregated particles with substantially uniform mean diameter. The particles are then separated from the solvent. The '454 Patent discourages forming particles in a crystalline state because during the precipitating procedure the crystal can dissolve and recrystallize thereby broadening the particle size distribution range. The '454 Patent encourages during the precipitating procedure to trap the particles in a metastable particle state.

United States Patent No. 5,605,785 discloses a process for nanomorphous dispersions of photographically useful compounds. The process of forming nanoamorphous dispersions includes any known process of emulsification that produces a disperse phase having amorphous particulates.

US-A-4,606,940 describes a process for the formation and concurrent encapsulation of small particles of an active compound. The process comprises adding, under stirring, a solution of an encapsulating material and an electrolyte in a second solvent to a solution of the active compound in a first solvent to precipitate the encapsulated active compound.

WO 99/16643 describes a composition for oral administration comprising a substantially pure crystalline form of the pharmaceutical active levosimendan. U5-A-5,122,543 describes an aqueous suspension comprising cubic or cuboidal crystals of carbamazepine having a size of from 10 to 200 $\mu$m.

## SUMMARY OF THE INVENTION

The present invention provides a method for preparing submicron particles of a pharmaceutically active compound according to claim 1. The term "organic compound" is sometimes used herein instead of "pharmaceutically active compound".

The present invention provides a method for preparing submicron sized particles of an organic compound, the solubility of which is greater in a water-miscible first solvent than in a second solvent which is aqueous. The process includes the steps of: (i) dissolving the organic compound in the water-miscible first solvent to form a solution, the first solvent being selected from the group consisting of N-methyl-2-pyrrolidinone, 2-pyrrolidone, dimethyl solfoxide, dimethylacetamide, lactic acid, methanol, ethanol, isopropanol, 3-pentanol, n-propanol, glycerol, butylene glycol, ethylene glycol, propylene glycol, mono- and diacylated monoglycerides, dimethyl isosorbide, acetone, dimethylformamide, 1,4-dioxane, polyethylene glycol, polyethylene glycol esters, polyethylene glycol sorbitans, polyethylene glycol monoalkyl ethers, polypropylene glycol, polypropylene alginate, PPG-10 butanediol, PPG-10 methyl glucose ether, PPG-20 methyl glucose ether, PPG-15 stearyl ether, propylene glycol dicaprylate, propylene glycol dicaprate, propylene glycol laurate; (ii) mixing the solution with the second solvent to define a pre-suspension; and (iii) adding energy to the pre-suspension to form particles having an average effective particle size of less than about 2 $\mu$m.

The present invention also provides a method for preparing submicron sized particles of an organic compound, the solubility of which is greater in a water-miscible first solvent than in a second solvent which is aqueous, the process comprising the steps of: (i) dissolving the organic compound in the water-miscible first solvent to form a solution, the first solvent being selected from the group consisting of N-methyl-2-pyrrolidinone, 2-pyrrolidone, dimethyl sulfoxide, dimethylacetamide, lactic acid, methanol, ethanol, isopropanol, 3-pentanol, n-propanol, glycerol, butylene glycol, ethylene glycol, propylene glycol, mono- and diacylated monoglycerides, dimethyl isosorbide, acetone, dimethylformamide, 1,4-dioxane, ethyl acetate, propyl acetate, polyethylene glycol, polyethylene glycol esters, polyethylene glycol sorbitans, polyethylene glycol monoalkyl ethers; polypropylene glycol, polypropylene alginate, PPG-10 butanediol, PPG-10 methyl glucose ether, PPG-20 methyl glucose ether, PPG-15 stearyl ether, propylene glycol dicaprylate, propylene glycol dicaprate, propylene glycollamate; (ii) mixing the solution with the second solvent to define a pre-suspension wherein the organic compound is in an amorphous form, a semicrystalline form or m a supercooled liquid form as determined by DSC and having an average effective particle size; and (III) annealing the pre-suspension to form particles having essentially the same average effective particle size of the pre-suspension and in a more stable form.

The present invention also provides a method for preparing submicron sized particles of an organic compound, the solubility of which is greater in a water-miscible first solvent than in a second solvent which is aqueous. The process includes the steps of: (i) dissolving the organic compound in the water-miscible first solvent to form a solution, the first solvent being selected from the group consisting of N-methyl-2-pyrrolidinone, 2-pyrrolidone, dimethyl sulfoxide, dimethylacetamide, lactic acid, methanol, ethanol, isopropanol, 3-pentanol, n-propanol, glycerol, butylene glycol, ethylene glycol, propylene glycol, mono- and diacylated monoglycerides, dimethyl isosorbide, acetone, dimethylformamide, 1,4-dioxane, polyethylene glycol, polyethylene glycol esters, polyethylene glycol sorbitans, polyethylene glycol monoalkyl ethers, polypropylene glycol, polypropylene alginate, PPG-10 butanediol, PPG-10 methyl glucose ether, PPG-20 methyl glucose ether, PPG-15 stearyl ether, propylene glycol dicaprylate, propylene glycol dicaprate, propylene glycol laurate; (ii) mixing the solution with the second solvent to define a pre-suspension of particles in a friable form; and (iii) adding energy to the pre-suspension to form particles having an average effective particle size of less than about 2 $\mu$m.

BRIEF DESCRIPTION OF THE DRAWINGS:

Not Applicable. The Drawings have been incorporated into the text.

DETAILED DESCRIPTION OF THE INVENTION:

There are described methods or processes for forming particles of an organic compound having an average effective particle size suitable for parenteral administration and preferably, the particle size is less than about 2 $\mu$m. The particles of the organic compound may also be in a form suitable for oral administration. Particles sizes for oral dosage forms can be in excess of 2 $\mu$m and typically less than about 7 $\mu$m. However, the particles can exceed 7 $\mu$m provided that the particles have sufficient bioavalability and other characteristics of an oral dosage form. Oral dosage forms include tablets, capsules, caplets, soft and hard gel capsules, or other delivery vehicle for delivering a drug by oral administration.

The processes can be separated into three general categories. Each of the categories of processes share the steps of: (1) dissolving an organic compound in a water miscible first organic solvent to create a first solution, (2) mixing the first solution with a second solvent of water to precipitate the organic compound to create a pre-suspension, and (3) adding energy to the presuspension in the form of high-shear mixing or heat to provide a stable form of the organic compound having the desired size ranges defined above.

The three categories of processes are distinguished based upon the physical properties of the organic compound as determined through x-ray diffraction studies, differential scanning calorimetry (DSC) studies or other suitable study conducted prior to the energy-addition step and after the energy-addition step. In the first process category, prior to the energy-addition step the organic compound in the presuspension takes an amorphous form, a semi-crystalline form or a supercooled liquid form and has an average effective particle size. After the energy-addition step the organic compound is in a crystalline form having an average effective particle size essentially the same as that of the presuspension (i.e., from less than about 2 $\mu$m).

In the second process category, prior to the energy-addition step the organic compound is in a crystalline form and has an average effective particle size. After the energy-addition step the organic compound is in a crystalline form having essentially the same average effective particle size as prior to the energy-addition step but the crystals after the energy-addition step are less likely to aggregate.

The lower tendency of the organic compound to aggregate is observed by laser dynamic light scattering and light microscopy.

In the third process category, prior to the energy-addition step the organic compound is in a crystalline form that is friable and has an average effective particle size. What is meant by the term "friable" is that the particles are fragile and are more easily broken down into smaller particles. After the energy-addition step the organic compound is in a crystalline form having an average effective particle size smaller than the crystals of the pre-suspension. By taking the steps necessary to place the organic compound in a crystalline form that is friable, the subsequent energy-addition step can be carried out more quickly and efficiently when compared to an organic compound in a less friable crystalline morphology.

The energy-addition step can be carried out in any fashion wherein the pre-suspension is exposed to cavitition, shearing or impact forces. The energy addition step may be an annealing step. Annealing is defined in this invention as the process of converting matter that is thermodynamically unstable into a more stable form by single or repeated application of energy (direct heat or mechanical stress), followed by thermal relaxation. This lowering of energy may be achieved by conversion of the solid form from a less ordered to a more ordered lattice structure. Alternatively, this stabilization may occur by a reordering of the surfactant molecules at the solid-liquid interface.

These three process categories will be discussed separately below. It should be understood, however, that the process conditions such as choice of surfactants or combination of surfactants, amount of surfactant used, temperature of reaction, rate of mixing of solutions, rate of precipitation and the like can be selected to allow for any drug to be processed under any one of the categories discussed next

The first process category, as well as the second and third process categories, can be further divided into two subcategories, Method A, and B shown diagramatically below.

**Figure 1: Method A:**

Drug + Water-miscible solvent → Concentrated drug solution → Water + surfactant(s) + optional buffer(s)

Heat and/or mechanical energy

Suspension of amorphous particles, semi-crystalline particles and/or supercooled liquid

Stable crystalline product ←

OPTIONAL STEPS

Separation of supernatant (by centrifugation, for example)

Solid residue → Diluent replenishment → High-shear mixing (e.g., homogenization) → Stabilized suspension

**Figure 2: Method B:**

Drug + solvent + surfactant(s) → Concentrated drug solution → Water + optional surfactant(s)

Heat and/or mechanical energy

Amorphous or semi-crystalline suspension

Stable crystalline product ←

OPTIONAL STEPS

Separation of supernatant (by centrifugation, for example)

Solid residue → Diluent replenishment → High-shear mixing (e.g., homogenization) → Stabilized suspension

A pharmaceutical compound as described herein may be any organic chemical entity whose solubility decreases from one solvent to another. The pharmaceutically active compound may be selected from various groups such as, but not limited to: antihyperlipidemics; antimicrobiab. e.g., antibacterials such as solfadiazine, antifungals such as itraconazole; non-steroidal anti-inflammatory drugs, e.g., indomethacin; antihypercholesteremic agents, e.g., probucol; and steroidal compounds, e.g., dexamethasone; immunosuppresants, e.g., cyclosporin A, tacrolimis, and mycophenolate mofetil. Or the organic compound might be from the group used as adjuvants or excipients in pharmaceutical preparations and cosmetics, such as, but not limited to, preservatives, e.g., propylparaben.

The first solvent is a solvent or mixture of solvents in which the organic compound of interest is relatively soluble and which is miscible with the second solvent. Examples of such solvents include, but are not limited to: polyvinylpyrrolidone, N-methyl-2-pyrrolidinone (also called N-methyl-2-pyrrolidone), 2-pyrrolidone, dimethyl sulfoxide, dimethylacetamide, lactic acid, methanol, ethanol, isopropanol, 3-pentanol, n-propanol, glycerol, butylene glycol (butanediol), ethylene glycol, propylene glycol, mono- and diacylated monoglycerides (such as glyceryl caprylate), dimethylisosorbide, acetone, dimethylformamide, 1,4-dioxane, polyethylene glycol (for example, PEG-4, PEG-8, PEG-9, PEG-12, PEG-14, PEG-16, PEG-120, PEG-75, PEG-150, polyethylene glycol esters (examples such as PEG-4 dilaurate, PEG-20 dilaurate, PEG-6 isostearate, PEG-8 palmitostearate, PEG-150 palmitostearate), polyethylene glycol sorbitans (such as PEG-20 sorbitan isostearate), polyethylene glycol monoalkyl ethers (examples such as PEG-3 dimethyl ether, PEG-4 dimethyl ether), polypropylene glycol (PPG), polypropylene alginate, PPG-10 butanediol, PPG-10 methyl glucose ether, PPG-20 methyl glucose ether, PPG-15 stearyl ether, propylene glycol dicaprylate/dicaprate, propylene glycol laurate.

**Method A.**

In Method A (see Figure 1), the organic compound ("drug") is first dissolved in the first solvent to create a first solution. The organic compound can be added from about 0.1% (w/v) to about 50% (w/v) depending on the solubility of the organic compound in the first solvent. Heating of the concentrate from about 30°C to about 100°C may be necessary to ensure total dissolution of the compound in the first solvent.

A second aqueous solution is provided with one or more optional surface modifiers such as an anionic surfactant, a cationic surfactant, a nonionic surfactant or a biological surface active molecule added thereto. Suitable anionic surfactants include but are not limited to potassium laurate, sodium lauryl sulfate, sodium dodecylsulfate, alkyl Polyoxyethylene sulfates, sodium alginate, dioctyl sodium sulfosuccinate, phosphatidyl choline, phosphatidyl glycerol, phosphatidyl inosine, phosphatidylserine, phosphatidic acid and their salts, glyceryl esters, sodium carboxymethylcellulose, cholic acid and other bile acids (e.g., cholic acid, deoxycholic acid, glycocholic acid, taurocholic acid, glycodeoxycholic acid) and salts thereof (e.g., sodium deoxycholate, etc.). Suitable cationic surfactants include but are not limited to quaternary ammonium compounds, such as benzalkonium chloride, cetyltrimethylammonium bromide, lauryldimethylbenzylammonium chloride, acyl carnitine hydrochlorides, or alkyl pyridinium halides. As anionic surfactants, phospholipids may be used. Suitable phospholipids include, for example phosphatidylcholine, phosphatidylethanolamine, phosphatidylserine, phosphatidylinositol, phosphatidylglycerol, phosphatidic acid, lysophospholipids, egg or soybean phospholipid or a combination thereof. The phospholipid may be salted or desalted, hydrogenated or partially hydrogenated or natural semisynthetic or synthetic.

Suitable nonionic surfactants include: polyoxyethylene fatty alcohol ethers (Macrogol and Brij), polyoxyethylene sorbitan fatty acid esters (Polysorbates), polyoxyethylene fatty acid esters (Myrj), sorbitan esters (Span), glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers (poloxomers), polaxamines, methylcellulose, hydroxycellulose, hydroxy propylcellulose, hydroxy propylmethylcellulose, noncrystalline cellulose, polysaccharides including starch and starch derivatives such as hydroxyethylstarch (HES), polyvinyl alcohol, and polyvinylpyrrolidone. In a preferred form of the invention, the nonionic surfactant is a polyoxyethylene and polyoxypropylene copolymer and preferably a block-copolymer of propylene glycol and ethylene glycol. Such polymers are sold under the tradename POLOXAMER also sometimes referred to as PLURONIC®, and sold by several suppliers including Spectrum Chemical and Ruger. Among polyoxyethylene fatty acid esters is included those having short alkyl chains. One example of such a surfactant is SOLUTOL® HS 15, polyethylene-660-hydroxystearate, manufactured by BASF Aktiengesellschaft.

Surface active biological molecules include such molecules as albumin, casein, heparin, hirudin or other appropriate proteins.

It may also be desirable to add a pH adjusting agent to the second solution such as sodium hydroxide, hydrochloric acid, tris buffer or citrate, acetate, lactate, meglumine, or the like. The second solution should have a pH within the range of from about 3 to about 11.

For oral dosage forms one or more of the following excipients may be utilized: gelatin, casein, lecithin (phosphatides), gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers, e.g., macrogol ethers such as cetomacrogol 1000, polyoxyethylene castor oil derivatives, polyoxyethylene sorbitan fatty acid esters, e.g., the commercially available Tweens.TM., polyethylene glycols, polyoxyethylene stearates, colloidol silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethylcellulose phthalate, noncrystalline cellulose, magnesium aluminum silicate, triethanolamine, polyvinyl alcohol (PVA), and polyvinylpyrrolidone (PVP). Most of these excipients are described in detail in the Handbook of Pharmaceutical Excipients, published jointly by the American Pharmaceutical Association and The Pharmaceutical Society of Great Britain, the Pharmaceutical Press, 1986. The surface modifiers are commercially available and/or can be prepared by techniques known in the art. Two or more surface modifiers can be used in combination.

Preferably, the method for preparing submicron sized particles of an organic compound includes the steps of adding the first solution to the second solution. The addition rate its dependent on the batch size, and precipitation kinetics for the organic compound. Typically, for a small-scale laboratory process (preparation of 1 liter), the addition rate is from about 0.05 cc per minute to about 10 cc per minute. During the addition, the solutions should be under constant agitation. It has been observed using light microscopy that amorphous particles, semi-crystalline solids, or a supercooled liquid are formed to create a pre-suspension. The method further includes the step of subjecting the pre-suspension to an annealing step to convert the amorphous particles, supercooled liquid or semicrystalline solid to a crystalline more stable solid state. The resulting particles will have an average effective particles size as measured by dynamic light scattering methods (e.g., photocorrelation spectroscopy, laser diffraction, low-angle laser light scattering (LALLS), medium-angle laser light scattering (MALLS), light obscuration methods (Coulter method, for example), rheology, or microscopy (light or electron) within the ranges set forth above).

The energy-addition step involves adding energy through sonication homogenization, counter current flow homogenization, microfluidization. The sample may be cooled or heated during this stage. The annealing step may be effected by a piston gap homogenizer such as the one sold by Avestin Inc. under the product designation Emulsi-Flex-C160. Alternatively, the annealing may be accomplished by nitrasonicanomising an ultrasonic processor such as the Vibra-Cell Ultrasonic Processor (600W), manufactured by Sonics and Materials, Inc. Still further the annealing may be accomplished by use of an emulsification apparams as described in U.S. Patent No.5,720,551.

Depending upon the rate of annealing, it may be desirable to adjust the temperature of the processed sample to within the range of from approximately -30°C to 30°C. Alternatively, in order to effect a desired phase change in the processed solid, it may also be necessary to heat the pre-suspension to a temperature within the range of from about 30°C to about 100°C during the annealing step.

**Method B**

Method B differs from Method A in the following respects. The first difference is a surfactant or combination of surfactants is added to the first solution. The surfactants may be selected from the groups of anionic, nonionic and cationinc surfactants set forth above.

Comparative Example of Method A and Method B and USPN 5,780,062

United States Patent No. 5,780,062 discloses a process for preparing small particles of an organic compound by first dissolving the compound in a suitable water-miscible first solvent. A second solution is prepared by dissolving a polymer and an amphiphile in aqueous solution. The first solution is then added to the second solution to form a precipitate that consists of the organic compound and a polymer-amphiphile complex. The '062 Patent does not disclose utilizing the energy-addition step of this invention in Methods A and B. Lack of stability is typically evidenced by rapid aggregation and particle growth. In some instances, amorphous particles recrystallize as large crystals. Adding energy to the pre-suspension in the manner disclosed above typically affords particles that show decreased rates of particle aggregation and growth, as well as the absence of recrystallization upon product storage.

Methods A and B are further distinguished from the process of the '062 patent by the absence of a step of forming a polymer-amphiphile complex prior to precipitation. In Method A, such a complex cannot be formed as no polymer is added to the diluent (aqueous) phase. In Method B, the surfactant, which may also act as an amphiphile, or polymer, is dissolved with the organic compound in the first solvent. This precludes the formation of any amphiphile-polymer complexes prior to precipitation. In the '062 Patent, successful precipitation of small particles relies upon the formation of an amphiphile-polymer complex prior to precipitation. The '062 Patent discloses the amphiphile-polymer complex forms aggregates in the aqueous second solution. The '062 Patent explains the hydrophobic organic compound interacts with the amphiphile-polymer complex, thereby reducing solubility of these aggregates and causing precipitation. In the present invention it has been demonstrated that the inclusion of the surfactant or polymer in the first solvent (Method B) leads, upon subsequent addition to second solvent, to formation of a more uniform, finer particulate than is afforded by the process outlined by the '062 Patent.

To this end, two formulations were prepared and analyzed. Each of the formulations have two solutions, a concentrate and an aqueous diluent, which are mixed together and then sonicated. The concentrate in each formulation has an organic compound (itraconazole), a water miscible solvent (N-methyl-2-pyrrolidinone or NMP) and possibly a polymer (poloxamer 188). The aqueous diluent has water, a tris buffer and possibly a polymer (poloxamer 188) and/or a surfactant (sodium deoxycholate). The average particle diameter of the organic particle is measured prior to sonication and after sonication.

The first formulation A has as the concentrate itraconazole and NMP. The aqueous diluent includes water, poloxamer 188, tris buffer and sodium deoxycholate. Thus the aqueous diluent includes a polymer (poloxamer 188), and an amphiphile (sodium deoxycholate), which may form a polymer/amphiphile complex, and, therefore, is in accordance with the disclosure of the '062 Patent (However, again the '062 Patent does not disclose an energy addition step.)

The second formulation B has as the concentrate itraconazole, NMP and poloxamer 188. The aqueous diluent includes water, tris buffer and sodium deoxycholate. This formulation is made in accordance with the present invention. Since the aqueous diluent does not contain a combination of a polymer (poloxamer) and an amphiphile (sodium deoxycholate), a polymer/amphiphile complex cannot form prior to the mixing step.

Table 1 shows the average particle diameters measured by laser diffraction on three replicate suspension preparations. An initial size determination was made, after which the sample was sonicated for 1 minute. The size determination was then repeated. The large size reduction upon sonication of Method A was indicative of particle aggregation.

Table 1:

| Method | Concentrate | Aqueous Diluent | Average particle diameter (microns) | After sonication (1 minute) |
|---|---|---|---|---|
| A | itraconazole (18%), N-methyl-2-pyrrolidinone (6 mL) | poloxamer 188 (2.3%), sodium deoxycholate (0.3%)tris buffer (5 mM, pH 8)water (qs to 94 mL) | 18.7 | 2.36 |
| | | | 10.7 | 2.46 |
| | | | 12.1 | 1.93 |
| B | itraconazole (18%) poloxamer 188 (37%) N-methyl-2-pyrrolidinone (6 mL) | sodium deoxycholate (0.3%)tris buffer (5 mM, pH 8)water (qs to 94 mL) | 0.194 | 0.198 |
| | | | 0.178 | 0.179 |
| | | | 0.181 | 0.177 |

A drug suspension resulting from application of the processes described in this invention may be administered directly as an injectable solution, provided Water for Injection is used in formulation and an appropriate means for solution sterilization is applied. Sterilization may be accomplished by separate sterilization of the drug concentrate (drug, solvent, and optional surfactant) and the diluent medium (water, and optional buffers and surfactants) prior to mixing to form the pre-suspension. Sterilization methods would include pre-filtration first through a 3.0 micron filter followed by filtration through a 0.45-micron particle filter, followed by steam or heat sterilization or sterile filtration through two redundant 0.2-micron membrane filters.

Optionally, a solvent-free suspension may be produced by solvent removal after precipitation. This can be accomplished by centrifugation, dialysis, diafiltration, force-field fractionation, high-pressure filtration or other separation techniques well known in the art. Complete removal of N-methyl-2-pyrrolidinone was typically carried out by one to three successive centrifugation runs; after each centrifugation (18,000 rpm for 30 minutes) the supernatant was decanted and discarded. A fresh volume of the suspension vehicle without the organic solvent was added to the remaining solids and the mixture was dispersed by homogenization. It will be recognized by others skilled in the art that other high-shear mixing techniques could be applied in this reconstitution step.

Furthermore, any undesired excipients such as surfactants may be replaced by a more desirable excipient by use of the separation methods described in the above paragraph. The solvent and first excipient may be discarded with the supernatant after centrifugation or filtration. A fresh volume of the suspension vehicle without the solvent and without the first excipient may then be added. Alternatively, a new surfactant may be added. For example, a suspension consisting of drug, N-methyl-2-pyrrolidinone (solvent), poloxamer 188 (first excipient), sodium deoxycholate, glycerol and water may be replaced with phospholipids (new surfactant), glycerol and water after centrifugation and removal of the supernatant.

I. First Process Category

The methods of the first process category generally include the step of dissolving the organic compound in a water miscible first solvent followed by the step of mixing this solution with an aqueous solution to form a presuspension wherein the organic compound is in an amorphous form, a semicrystalline form or in a supercooled liquid form as determined by x-ray diffraction studies, DSC, light microscopy or other analytical techniques and has an average effective particle size within one of the effective particle size ranges set forth above. The mixing step is followed by an energy-addition step and, in a preferred form of the invention an annealing step.

II. Second Process Category

The methods of the second processes category include essentially the same steps as in the steps of the first processes category but differ in the following respect. An x-ray diffraction, DSC or other suitable analytical techniques of the presuspension shows the organic compound in a crystalline form and having an average effective particle size. The organic compound after the energy-addition step has essentially the same average effective particle size as prior to the energy-addition step but has less of a tendency to aggregate into larger particles when compared to that of the particles of the presuspension. Without being bound to a theory, it is believed the differences in the particle stability may be due to a reordering of the surfactant molecules at the solid-liquid interface.

III Third Process Category

The methods of the third category modify the first two steps of those of the first and second processes categories to ensure the organic compound in the presuspension is in a friable form having an average effective particle size (e.g., such as slender needles and thin plates). Friable particles can be formed by selecting suitable solvents, surfactants or combination of surfactants, the temperature of the individual solutions, the rate of mixing and rate of precipitation and the like. Friability may also be enhanced by the introduction of lattice defects (e.g., cleavage planes) during the steps of mixing the first solution with the aqueons solution. This would arise by rapid crytallization such as that afforded in the precipitation step. In the energy-addition step these friable crystals are converted to crystals that are kinetically stabilized and having an average effective particle size smaller than those of the presuspension. Kinetically stabilized means particles have a reduced tendency to aggregate when compared to particles that are not kinetically stabilized. In such instance the energy-addition step results in a breaking up of the friable particles. By ensuring the particles of the presuspension are in a friable state, the organic compound can more easily and more quickly be prepared into a particle within the desired size ranges when compared to processing an organic compound where the steps have not been taken to render it in a friable form.

**Polymorph Control**

The process may comprise additional steps for controlling the crystal structure of the pharmaceutically-active compound to ultimately produce a suspension of the compound in the desired size range and a desired crystal structure. What is meant by the term "crystal structure" is the arrangement of the atoms within the unit cell of the crystal. Pharmaceutically-active compounds that can be crystallized into different crystal structures are said to be polymorphic. Identification of polymorphs is important step in drug formulation since different polymorphs of the same drug can show differences in solubility, therapentic activity, bioavailabilty, and suspension stability. Accordingly, it is important to control the polymorphic form of the compound for ensuring product purity and batch-to-batch reproducibility.

The steps to control the polymorphic form of the compound includes seeding the first solution, the second solvent or the presuspension to ensure the formation of the desired polymorph. Seeding includes using a seed compound or adding energy. Preferably, the seed compound is the pharmaceutically-active compound in the desired polymorphic form. Alternatively, the seed compound can also be an inert impurity or an organic compound with a structure similar to that of the desired polymorph such as a bile salt

The seed compound can be precipitated from the first solution. This method includes the steps of adding the pharmaceutically-active compound in sufficient quantity to exceed the solubility of the pharmaceutically-active compound in the first solvent to create a supersaturated solution. The supersaturated solution is treated to precipitate the pharmaceutically-active compound in the desired polymorphic form. Treafing the supersaturated solution includes aging the solution for a time period until the formation of a crystal or crystals is observed to create a seeding mixture. It is also possible to add energy to the supersaturated solution to cause the pharmaceutically-active compound to precipitate out of the solution in the desired polymorph. The energy can be added in a variety of ways including the energy addition steps described above. Further energy can be added by heating or exposing the presuspension to electromagnetic energy, particle beam, or electron beam sources. The electromagnetic energy includes using a laser beam, dynamic electromagnetic energy, or other radiation sources. It is further contemplated utilizing ultrasound, static electric field and a static magnetic field as the energy addition source.

Preferably, the method for producing seed crystals from an aged supersaturated solution includes the steps of: (i) adding a quantity of the pharmaceutically-active compound to the first organic solvent to create a supersaturated solution; (ii) aging the supersaturated solution to form detectable crystals to create a seeding mixture; and (iii) mixing the seeding mixture with the second solvent to precipitate the pharmaceutically-active compound to create a presuspension. The presuspension can then be further processed as described in detail above to provide an aqueous suspension of the pharmaceutically-active compound in the desired polymorph and in the desired size range.

Seeding can also be accomplished by adding energy to the first solution, the second solvent or the presuspension provided that the exposed liquid or liquids contain the pharmaceutically active compound or a seed material. The energy can be added in the same fashion as described above for the supersaturated solution.

Accordingly, there is described a composition of matter of a pharmaceutically active compound in a desired polymorphic form essentially free of the unspecified polymorph or polymorphs. One such example is set forth in example 16 below where seeding during microprecipitation provides a polymorph of itraconazole essentially free of the polymorph of the raw material. It is contemplated the methods of this invention can apply used to selectively produce a desired polymorph for numerous pharmaceutically active compounds.

Examples

Examples of Process Category 1

Example 1: Preparation of itraconazole suspension by use of Process Category 1, Method A with homogenization.

To a 3-L flask add 1680 mL of Water for Injection. Heat liquid to 60-65°C, and then slowly add 44 grams of Pluronic F-68 (poloxamer 188), and 12 grams of sodium deoxycholate, stirring after each addition to dissolve the solids. After addition of solids is complete, stir for another 15 minutes at 60-65°C to ensure complete dissolution. Prepare a 50 mM tris (tromethamine) buffer by dissolving 6.06 grams of tris in 800 mL of Water for Injection. Titrate this solution to pH 8.0 with 0.1 M hydrochloric acid. Dilute the resulting solution to 1 liter with additional Water for Injection. Add 200 mL of the tris buffer to the poloxamer/deoxycholate solution. Stir thoroughly to mix solutions.

In a 150-mL beaker add 20 grams of itraconazole and 120 mL of N-methyl-2-pyrrolidinone. Heat mixture to 50-60°C, and stir to dissolve solids. After total dissolution is visually apparent, stir another 15 minutes to ensure complete dissolution. Cool itraconazole-NMP solution to room temperature.

Charge a syringe pump (two 60-mL glass syringes) with the 120-mL of itraconazole solution prepared previously. Meanwhile pour all of the surfactant solution into a homogenizer hopper which has been cooled to 0-5°C (this may either by accomplished by use of a jacketed hopper through which refrigerant is circulated, or by surrounding the hopper with ice). Position a mechanical stirrer into the surfactant solution so that the blades are fully immersed. Using the syringe pump, slowly (1-3 mL/min) add all of the itraconazole solution to the stirred, cooled surfactant solution. A stirring rate of at least 700 rpm is recommended. An aliquot of the resulting suspension (Suspension A) is analyzed by light microscopy (Hoffman Modulation Contrast) and by laser diffraction (Horiba). Suspension A is observed by light microscopy to consist of roughly spherical amorphous particles (under 1 micron), either bound to each other in aggregates or freely moving by Brownian motion. See Figure 3. Dynamic light scattering measurements typically afford a bimodal distribution pattern signifying the presence of aggregates (10-100 microns in size) and the presence of single amorphous particles ranging 200-700 nm in median particle diameter.

The suspension is immediately homogenized (at 10,000 to 30,000 psi) for 10-30 minutes. At the end of homogenization, the temperature of the suspension in the hopper does not exceed 75°C. The homogenized suspension is collected in 500-mL bottles, which are cooled immediately in the refrigerator (2-8°C). This suspension (Suspension B) is analyzed by light microscopy and is found to consist of small elongated plates with a length of 0.5 to 2 microns and a width in the 0.2-1 micron range. See Figure 4. Dynamic light scattering measurements typically indicate a median diameter of 200-700 nm.

**Figure 3: Amorphous particles prior to homogenization (Example 1).**

**Figure 4: Particles after annealing by homogenization.**

<u>Stability of Suspension A ("Pre-suspension") (Example 1)</u>

During microscopic examination of the aliquot of Suspension A, crystallization of the amorphous solid was directly observed. Suspension A was stored at 2-8 °C for 12 hours and examined by light microscopy. Gross visual inspection of the sample revealed severe flocculation, with some of the contents settling to the bottom of the container. Microscopic examination indicated the presence of large, elongated, plate-like crystals over 10 microns in length.

Stability of Suspension B

As opposed to the instability of Suspension A, Suspension B was stable at 2-8°C for the duration of the preliminary stability study (1 month). Microscopy on the aged sample clearly demonstrated that no significant change in the morphology or size of the particles had occurred. This was confirmed by light scattering measurement.

Example 2: Preparation of itraconazole suspension by use of Process Category 1, Method A with ultrasonication.

To a 500-mL stainless steel vessel add 252 mL of Water for Injection. Heat liquid to 60-65°C, and then slowly add 6.6 grams of Pluronic F-68 (poloxamer 188), and 0.9 grams of sodium deoxycholate, stirring after each addition to dissolve the solids. After addition of solids is complete, stir for another 15 minutes at 60-65°C to ensure complete dissolution. Prepare a 50 mM tris (tromethamine) buffer by dissolving 6.06 grams of tris in 800 mL of Water for Injection. Titrate this solution to pH 8.0 with 0.1 M hydrochloric acid. Dilute the resulting solution to 1 liter with additional Water for Injection. Add 30 mL of the tris buffer to the poloxamer/deoxycholate solution. Stir thoroughly to mix solutions.

In a 30-mL container add 3 grams of itraconazole and 18 mL of N-methyl-2-pyrrolidinone. Heat mixture to 50-60°C, and stir to dissolve solids. After total dissolution is visually apparent, stir another 15 minutes to ensure complete dissolution. Cool itraconazole-NMP solution to room temperature.

Charge a syringe pump with 18-mL of itraconazole solution prepared in a previous step. Position a mechanical stirrer into the surfactant solution so that the blades are fully immersed. Cool the container to 0-5°C by immersion in an ice bath. Using the syringe pump, slowly (1-3 mL/min) add all of the itraconazole solution to the stirred, cooled surfactant solution. A stirring rate of at least 700 rpm is recommended. Immerse an ultrasonicator horn in the resulting suspension so that the probe is approximately 1 cm above the bottom of the stainless steel vessel. Sonicate (10,000 to 25,000 Hz, at least 400W) for 15 to 20 minute in 5-minute intervals. After the first 5-minute sonication, remove the ice bath and proceed with further sonication. At the end of ultrasonication, the temperature of the suspension in the vessel does not exceed 75°C.

The suspension is collected in a 500-mL Type I glass bottle, which is cooled immediately in the refrigerator (2-8°C). Characteristics of particle morphology of the suspension before and after sonication were very similar to that seen in Method A before and after homogenization (see Example 1).

Example 3: Preparation of itraconazole suspension by use of Process Category I, Method B with homogenization.

Prepare a 50 mM tris (tromethamine) buffer by dissolving 6.06 grams of tris in 800 mL of Water for Injection. Titrate this solution to pH 8.0 with 0.1 M hydrochloric acid. Dilute the resulting solution to 1 liter with additional Water for Injection. To a 3-L flask add 1680 mL of Water for Injection. Add 200 mL of the tris buffer to the 1680 mL of water. Stir thoroughly to mix solutions.

In a 150-mL beaker add 44 grams of Pluronic F-68 (poloxamer 188) and 12 grams of sodium deoxycholate to 120 mL of N-methyl-2-pyrrolidinone. Heat the mixture to 50-60°C, and stir to dissolve solids. After total dissolution is visually apparent, stir another 15 minutes to ensure complete dissolution. To this solution, add 20 grams of itraconazole, and stir until totally dissolved. Cool the itraconazole-surfactant-NMP solution to room temperature.

Charge a syringe pump (two 60-mL glass syringes) with the 120-mL of the concentrated itraconazole solution prepared previously. Meanwhile pour the diluted tris buffer solution prepared above into a homogenizer hopper which has been cooled to 0-5°C (this may either by accomplished by use of a jacketed hopper through which refrigerant is circulated, or by surrounding the hopper with ice). Position a mechanical stirrer into the buffer solution so that the blades are fully immersed. Using the syringe pump, slowly (1-3 mL/min) add all of the itraconazole-surfactant concentrate to the stirred, cooled buffer solution. A stirring rate of at least 700 rpm is recommended. The resulting cooled suspension is immediately homogenized (at 10,000 to 30,000 psi) for 10-30 minutes. At the end of homogenization, the temperature of the suspension in the hopper does not exceed 75°C.

The homogenized suspension is collected in 500-mL bottles, which are cooled immediately in the refrigerator (2-8°C). Characteristics of particle morphology of the suspension before and after homogenization were very similar to that seen in Example 1, except that in process category 1B, the pre-homogenized material tended to form fewer and smaller aggregates which resulted in a much smaller overall particle size as measured by laser diffraction. After homogenization, dynamic light scattering results were typically identical to those presented in Example 1.

Example 4: Preparation of itraconazole suspension by use of Process Category 1, Method B with ultrasonication.

To a 500-mL flask add 252 mL of Water for Injection. Prepare a 50 mM tris (tromethamine) buffer by dissolving 6.06 grams of tris in 800 mL of Water for Injection. Titrate this solution to pH 8.0 with 0.1 M hydrochloric acid. Dilute the resulting solution to I liter with additional Water for Injection. Add 30 mL of the tris buffer to the water. Stir thoroughly to mix solutions.

In a 30-mL beaker add 6.6 grams of Pluronic F-68 (poloxamer 188) and 0.9 grams of sodium deoxycholate to 18 mL of N-methyl-2-pyrrolidinone. Heat the mixture to 50-60°C, and stir to dissolve solids. After total dissolution is visually apparent, stir another 15 minutes to ensure complete dissolution. To this solution, add 3.0 grams of itraconazole, and stir until totally dissolved. Cool the itraconazole-surfactant-NMP solution to room temperature.

Charge a syringe pump (one 30-mL glass syringe with the 18-mL of the concentrated itraconazole solution prepared previously. Position a mechanical stirrer into the buffer solution so that the blades are fully immersed. Cool the container to 0-5°C by immersion in an ice bath. Using the syringe pump, slowly (1-3 mL/min) add all of the itraconazole-surfactant concentrate to the stirred, cooled buffer solution. A stirring rate of at least 700 rpm is recommended. The resulting cooled suspension is immediately sonicated (10,000 to 25,000 Hz, at least 400 W) for 15-20 minutes, in 5-minute intervals. After the first 5-minute sonication, remove the ice bath and proceed with further sonication. At the end of ultrasonication, the temperature of the suspension in the hopper does not exceed 75°C.

The resultant suspension is collected in a 500-mL bottle, which is cooled immediately in the refrigerator (2-8°C). Characteristics of particle morphology of the suspension before and after sonication were very similar to that seen in Example 1, except that in Process Category 1, Method B, the pre-sonicated material tended to form fewer and smaller aggregates which resulted in a much smaller overall particle size as measured by laser diffraction. After ultrasonication, dynamic light scattering results were typically identical to those presented in Example 1.

B. Examples of Process Category 2

Example 5: Preparation of itraconazole suspension (1%) with 0.75% Solutol® HR (PEG-660 12-hydroxystearate) Process Category 2, Method B.

Solutol (2.25 g) and itraconazole (3.0 g) were weighed into a beaker and 36 mL of filtered N-methyl-2-pyrrolidinone (NMP) was added. This mixture was stirred under low heat (up to 40°C) for approximately 15 minutes until the solution ingredients were dissolved. The solution was cooled to room temperature and was filtered through a 0.2-micron filter under vacuum. Two 60-mL syringes were filled with the filtered drug concentrate and were placed in a syringe pump. The pump was set to deliver approximately 1 mL/min of concentrate to a rapidly stirred (400 rpm) aqueous buffer solution. The buffer solution consisted of 22 g/L of glycerol in 5 mM tris buffer. Throughout concentrate addition, the buffer solution was kept in an ice bath at 2-3°C. At the end of the precipitation, after complete addition of concentrate to the buffer solution, about 100 mL of the suspension was centrifuged for 1 hour, the supernatant was discarded. The precipitate was resuspended in a 20% NMP solution in water, and again centrifuged for 1 hour. The material was dried overnight in a vacuum oven at 25°C. The dried material was transferred to a vial and analyzed by X-ray diffractometry using chromium radiation (see Figure 5).

Another 100 mL-aliquot of the microprecipitated suspension was sonicated for 30 minutes at 20,000 Hz, 80% full amplitude (full amplitude = 600 W). The sonicated sample was homogenized in 3 equal aliquots each for 45 minutes (Avestin C5, 2-5°C, 15,000-20,000 psi). The combined fractions were centrifuged for about 3 hours, the supernatant removed, and the precipitate resuspended in 20% NMP. The resuspended mixture was centrifuged again (15,000 rpm at 5°C). The supernatant was decanted off and the precipitate was vacuum dried overnight at 25°C. The precipitate was submitted for analysis by X-ray diffractometry (see Figure 5). As seen in Figure 5, the X-ray diffraction patterns of processed samples, before and after homogenization, are essentially identical, yet show a significantly different pattern as compared with the starting raw material. The unhomogenized suspension is unstable and agglomerates upon storage at room temperature. The stabilization that occurs as a result of homogenization is believed to arise from rearrangement of surfactant on the surface of the particle. This rearrangement should result in a lower propensity for particle aggregation.

Figure 5: X-Ray diffractogram of microprecipitated itraconazole with polyethylene glycol-660 12-hydroxystearate before and after homogenization (Example 5).

C. Examples of Process Category

Example 6: Preparation of carbamazepine suspension by use of Process Category 3, Method A with homogenization.

2.08 g of carbamazepine was dissolved into 10 mL of NMP. 1.0 mL of this concentrate was subsequently dripped at 0.1 mL/min into 20 mL of a stirred solution of 1.2% lecithin and 2.25% glycerin. The temperature of the lecithin system was held at 2-5°C during the entire addition. The predispersion was next homogenized cold (5-15°C) for 35 minutes at 15,000 psi. The pressure was increased to 23,000 psi and the homogenization was continued for another 20 minutes. The particles produced by the process had a mean diameter of 0.881 $\mu$m with 99% of the particles being less than 2.44 um.

Example 7: Preparation of 1% carbamazepine suspension with 0.125% Solutol® by use of Process Category 3, Method B with homogenization.

A drug concentrate of 20% carbamazepine and 5% glycodeoxycholic acid (Sigma Chemical Co.) in N-methyl-2-pyrrolidinone was prepared. The microprecipitation step involved adding the drug concentrate to the receiving solution (distilled water) at a rate of 0.1 mL/min. The receiving solution was stirred and maintained at approximately 5° C during precipitation. After precipitation, the final ingredient concentrations were 1 % carbamazepine and 0.125% Solutol®. The drug crystals were examined under a light microscope using positive phase contrast (400X). The precipitate consisted of fine needles approximately 2 microns in diameter and ranging from 50 -150 microns in length.

Homogenization (Avestin C-50 piston-gap homogenizer) at approximately 20,000 psi for approximately 15 minutes results in small particles, less than 1 micron in size and largely unaggregated. Laser diffraction analysis (Horiba) of the homogenized material showed that the particles had a mean size of 0.4 micron with 99% of the particles less than 0.8 micron. Low energy sonication, suitable for breaking agglomerated particles, but not with sufficient energy to cause a cominution of individual particles, of the sample before Horiba analysis had no effect on the results (numbers were the same with and without sonication). This result was consistent with the absence of particle agglomeration.

Samples prepared by the above process were centrifuged and the supernatant solutions replaced with a replacement solution consisting of 0.125% Solutol®. After centrifugation and supernatant replacement, the suspension ingredient concentrations were 1% carbamazepine and 0.125% Solutol®. The samples were re-homogenized by piston-gap homogenizer and stored at 5°C. After 4 weeks storage, the suspension had a mean particle size of 0.751 with 99% less than 1.729. Numbers reported are from Horiba analysis on unsonicated samples.

Example 8: Preparation of 1% carbamazepine suspension with 0.06% sodium glycodeoxycholate and 0.06% poloxamer 188 by use of Process Category 3, Method B with homogenization.

A drug concentrate comprising 20% carbamazepine and 5% glycodeoxycholate in N-methyl-2-pyrrolidinone was prepared. The microprecipitation step involved adding the drug concentrate to the receiving solution (distilled water) at a rate of 0.1 mL/min. Thus, this and the following examples demonstrate that adding a surfactant or other excipient to the aqueous precipitating solution in Methods A and B above is optional. The receiving solution was stirred and maintained at approximately 5° C during precipitation. After precipitation, the final ingredient concentrations were 1% carbamazepine and 0.125% Solutol®. The drug crystals were examined under a light microscope using positive phase contrast (400X). The precipitate consisted of fine needles approximately 2 microns in diameter and ranging from 50 - 150 microns in length. Comparison of the precipitate with the raw material before precipitation reveals that the precipitation step in the presence of surface modifier (glycodeoxycholic acid) results in very slender crystals that are much thinner than the starting raw material (see Figure 6).

Homogenization (Avestin C-50 piston-gap homogenizer) at approximately 20,000 psi for approximately 15 minutes results in small particles, less than 1 micron in size and largely unaggregated. See Figure 7. Laser diffraction analysis (Horiba) of the homogenized material showed that the particles had a mean size of 0.4 micron with 99% of the particles less than 0.8 micron. Sonication of the sample before Horiba analysis had no effect on the results (numbers were the same with and without sonication). This result was consistent with the absence of particle agglomeration.

Samples prepared by the above process were centrifuged and the supernatant solutions replaced with a replacement solution consisting of 0.06% glycodeoxycholic acid (Sigma Chemical Co.) and 0.06% Poloxamer 188. The samples were re-homogenized by piston-gap homogenizer and stored at 5° C. After 2 weeks storage, the suspension had a mean particle size of 0.531 micron with 99% less than 1.14 micron. Numbers reported are from Horiba analysis on unsonicated samples.

Figure 6: Carbamazepine crystals before homogenization (Example 6).

| Raw material, before precipitation | After precipitation |
|---|---|

Figure 7: Carbamazepine microparticulate after homogenization (Avestin C-50)

(Example 6).

Mathematical Analysis (Example 8) of force required to break precipitated particles as compared to force required to break particles of the starting raw material (carbamazepine):

The width of the largest crystals seen in the carbamazepine raw material (Figure 6, picture on left) are roughly 10-fold greater than the width of crystals in the microprecipitated material (Figure 6, picture on right). On the assumption that the ratio of crystal thickness (1:10) is proportional to the ratio of crystal width (1:10), then the moment of force required to cleave the larger crystal in the raw material should be approximately 1,000-times greater than the force needed to break the microprecipitated material, since:

$$e_L = 6PL/(Ewx^4) \qquad\qquad Eq. 1$$

where,

$e_L$ = longitudinal strain required to break the crystal ("yield value")

P = load on beam
L = distance from load to fulcrum
E = elasticity modulus
w = width of crystal
x = thickness of crystal

Let us assume that L and E are the same for the raw material and the precipitated material. Additionally, let us assume that $w/w_0 = x/x_0 = 10$. Then,

$$(e_L)_0 = 6P_0L/(Ew_0x_0{}^2),$$

where the '0' subscripts refer to raw material

$$e_L = 6PL/(Ewx^2),$$

for the microprecipitate
Equating $(e_L)_0$ and $e_L$,

$$6PL/(Ewx^2) = 6P_0L/(Ew_0x_0{}^2)$$

After simplification,

$$P = P_0 (w/w_0) (x/x_0)^2 = P_0 (0.1) (0.1)^2 = 0.001 P_0$$

Thus, the yield force, P, required to break the microprecipitated solid is one-thousandth the required force necessary to break the starting crystalline solid. If, because of rapid precipitation, lattice defects or amorphic properties are introduced, then the modulus (E) should decrease, making the microprecipitate even easier to cleave.

Example 9: Preparation of 1.6% (w/v) prednisolone suspension with 0.05% sodium deoxycholate and 3% N-methyl-2-pyrrolidinone Process Category 3, Method B

A schematic of the overall manufacturing process is presented in Figure 8. A concentrated solution of prednisolone and sodium deoxycholate was prepared. Prednisolone (32g) and sodium deoxycholate (1g) were added to a sufficient volume of 1-methyl 2-pyrrolidinone (NMP) to produce a final volume of 60 mL. The resulting prednisolone concentration was approximately 533.3 mg/mL and the sodium deoxycholate concentration was approximately 16.67 mg/mL. 60mL of NMP concentrate was added to 2 L of water cooled to 5°C at an addition rate of 2.5 mL/min while stirring at approximately 400 rpm. The resulting suspension contained slender needle-shaped crystals less than 2 $\mu$m in width (Figure 9). The concentration contained in the precipitated suspension was 1.6% (w/v) prednisolone, 0.05% sodium deoxycholate, and 3% NMP.

The precipitated suspension was pH adjusted to 7.5-8.5 using sodium hydroxide and hydrochloric acid then homogenized (Avestin C-50 piston-gap homogenizer) for 10 passes at 10,000 psi. The NMP was removed by performing 2 successive centrifugation steps replacing the supernatant each time with a fresh surfactant solution, which contained the desired concentrations of surfactants needed to stabilize the suspension (see Table 2). The suspension was homogenized for another 10 passes at 10,000 psi. The final suspension contained particles with a mean particle size of less than 1 $\mu$m, and 99% of particles less than 2 $\mu$m. Figure 10 is a photomicrograph of the final prednisolone suspension after homogenization.

A variety of different surfactants at varying concentrations were used in the centrifugation/surfactant replacement step (see Table 2). Table 2 lists combinations of surfactants that were stable with respect to particle size (mean < 1 $\mu$m, 99%< 2 $\mu$m), pH (6-8), drug concentration (less than 2% loss) and re-suspendability (resuspended in 60 seconds or less).

## Figure 8: Diagram of Microprecipitation Process for Prednisolone (Examples 9-12)

Notably this process allows for adding the active compound to an aqueous diluent without the presence of a surfactant or other additive. This is a modification of process Method B in Figure 2.

## Figure 9: Photomicrograph of prednisolone suspension before homogenization (Hoffman Modulation Contrast, 1250X magnification)

Figure 10: Photomicrograph of prednisolone suspension after homogenization (Hoffman Modulation Contrast, 1250X magnification).

Table 2: List of stable prednisolone suspensions prepared by microprecipitation process of Figure 8 (Example 9)

| Formulation | | | 2 Weeks | | 2 Months | | | | | | |
| --- | --- | --- | --- | --- | --- | --- | --- | --- | --- | --- | --- |
| | Initial | | 40°C | | 5°C | | 25°C | | 40°C | | |
| | Mean | >99% | Mean | >99% | Mean | >99% | Mean | >99% | Mean | >99% | % Loss* |
| 1.6% prednisolone, 0.6% phospholipids, 0.5% sodium deoxycholate, 5 mM TRIS, 2.2% glycerol ** | 0.79 | 1.65 | 0.84 | 1.79 | 0.83 | 1.86. | 0.82 | 1.78 | 0.82 | 1.93 | <2% |
| 1.6% prednisolone, 0.6% Solutol®, 0.5% sodium deoxycholate, 2.2% glycerol | 0.77 | 1.52 | 0.79 | 1.67 | 0.805 | 1.763 | 0.796 | 1.693 | 0.81 | 1.633 | <2% |
| 1.6% prednisolone, 0.1% poloxamer 188, 0.5% sodium deoxycholate, 2.2% glycerol | 0.64 | 1.16 | 0.82 | 1.78 | 0.696 | 1.385 | 0.758 | 1.698 | 0.719 | 1.473 | <2% |
| 1.6% prednisolone, 5% phospholipids, 5 mM TRIS, 2.2% glycerol | 0.824 | 1.77 | 0.87 | 1.93 | 0.88 | 1.95 | 0.869 | 1.778 | 0.909 | 1.993 | <2% |
| * Difference in itraconazole concentration between samples stored for 2 months at 5 and 25°C.<br>** Stable through at least 6 months.<br>Particle sizes (by laser light scattering), in microns:<br>5°C: 0.80 (mean), 1.7 (99%)<br>25°C: 0.90 (mean); 2.51 (99%)<br>40°C: 0.99 (mean); 2.03 (99%)<br>Difference in itraconazole concentration between samples stored at 5 and 25°C: <2% | | | | | | | | | | | |

Example 10: Preparation of prednisolone suspension by use of Process Category 3, Method A with homogenization.

32 g of prednisolone was dissolved into 40 mL of NMP. Gentle heating at 40-50°C was required to effect dissolution. The drug NMP concentrate was subsequently dripped at 2.5 mL/min into 2 liters of a stirred solution that consisted of 0.1.2% lecithin and 2.2% glycerin. No other surface modifiers were added. The surfactant system was buffered at pH = 8.0 with 5 mM tris buffer and the temperature was held at 0° to 5°C during the entire precipitation process. The post-precipitated dispersion was next homogenized cold (5-15 °C) for 20 passes at 10,000 psi. Following homogenization, the NMP was removed by centrifuging the suspension, removing the supernatant, and replacing the supernatant with fresh surfactant solution. This post-centrifuged suspension was then rehomogenized cold (5-15 °C) for another 20 passes at 10,000 psi. The particles produced by this process had a mean diameter of 0.927 um with 99% of the particles being less than 2.36 um.

Example 11: Preparation of nabumetone suspension by use of Process Category 3, Method B with homogenization.

Surfactant (2.2 g of poloxamer 188) was dissolved in 6 mL of N-methyl-2-pyrrolidinone. This solution was stirred at 45°C for 15 minutes, after which 1.0 g of nabumetone was added. The drug dissolved rapidly. Diluent was prepared which consisted of 5 mM tris buffer with 2.2% glycerol, and adjusted to pH 8. A 100-mL portion of diluent was cooled in an ice bath. The drug concentrate was slowly added (approximately 0.8 mL/min) to the diluent with vigorous stirring. This crude suspension was homogenized at 15,000 psi for 30 minutes and then at 20,000 psi for 30 minutes (temperature = 5°C). The final nanosuspension was found to be 930 nm in effective mean diameter (analyzed by laser diffraction). 99% of the particles were less than approximately 2.6 microns.

Example 12: Preparation of nabumetone suspension by use of Process Category 3, Method B with homogenization and the use of Solutol® HS 15 as the surfactant. Replacement of supernatant liquid with a phospholipid medium.

Nabumetone (0.987 grams) was dissolved in 8 mL of N-methyl-2-pyrrolidinone. To this solution was added 2.2 grams of Solutol® HS 15. This mixture was stirred until complete dissolution of the surfactant in the drug concentrate. Diluent was prepared, which consisted of 5 mM tris buffer with 2.2% glycerol, and which was adjusted to pH 8. The diluent was cooled in an ice bath, and the drug concentrate was slowly added (approximately 0.5 mL/min) to the diluent with vigorous stirring. This crude suspension was homogenized for 20 minutes at 15,000 psi, and for 30 minutes at 20,000 psi.
The suspension was centrifuged at 15,000 rpm for 15 minutes and the supernatant was removed and discarded. The remaining solid pellet was resuspended in a diluent consisting of 1.2% phospholipids. This medium was equal in volume to the amount of supernatant removed in the previous step. The resulting suspension was then homogenized at approximately 21,000 psi for 30 minutes. The final suspension was analyzed by laser diffraction and was found to contain particles with a mean diameter of 542 nm, and a 99% cumulative particle distribution sized less than 1 micron.

Example 13: Preparation of 1% itraconazole suspension with poloxamer with particles of a mean diameter of approximately 220 nm

Itraconazole concentrate was prepared by dissolving 10.02 grams of itraconazole in 60 mL of N-methyl-2-pyrrolidinone. Heating to 70°C was required to dissolve the drug. The solution was then cooled to room temperature. A portion of 50 mM tris(hydroxymethyl)aminomethane buffer (tris buffer) was prepared and was pH adjusted to 8.0 with 5M hydrochloric acid. An aqueous surfactant solution was prepared by combining 22 g/L poloxamer 407, 3.0 g/L egg phosphatides, 22g/L glycerol, and 3.0 g/L sodium cholate dihydrate. 900 mL of the surfactant solution was mixed with 100 mL of the tris buffer to provide 1000 mL of aqueous diluent.
The aqueous diluent was added to the hopper of the homogenizer (APV Gaulin Model 15MR-8TA), which was cooled by using an ice jacket. The solution was rapidly stirred (4700 rpm) and the temperature was monitored. The itraconazole concentrate was slowly added, by use of a syringe pump, at a rate of approximately 2 mL/min. Addition was complete after approximately 30 minute. The resulting suspension was stirred for another 30 minutes while the hopper was still being cooled in an ice jacket, and an aliquot was removed for analysis by light microscopy any dynamic light scatting. The remaining suspension was subsequently homogenized for 15 minutes at 10,000 psi. By the end of the homogenization the temperature had risen to 74°C. The homogenized suspension was collected in a 1-L Type I glass bottle and sealed with a rubber closure. The bottle containing suspension was stored in a refrigerator at 5°C.
A sample of the suspension before homogenization showed the sample to consist of both free particles, clumps of particles, and multilamellar lipid bodies. The free particles could not be clearly visualized due to Brownian motion; however, many of the aggregates appeared to consist of amorphous, non-crystalline material.
The homogenized sample contained free submicron particles having excellent size homogeneity without visible lipid vesicles. Dynamic light scattering showed a monodisperse logarithmic size distribution with a median diameter of approximately 220 nm. The upper 99% cumulative size cutoff was approximately 500 nm. Figure 11 shows a comparison of the size distribution of the prepared nanosuspension with that of a typical parenteral fat emulsion product (10% Intralipid®, Pharmacia).

Figure 11: Comparison of size distributions of nanosuspensions (this invention) and commercial fat emulsion. (Example 13)

Example 14: Preparation of 1% itraconazole nanosuspension with hydroxyethylstarch

Preparation of Solution A: Hydroxyethylstarch (1 g, Ajinomoto) was dissolved in 3 mL of N-methyl-2-pyrrolidinone (NMP). This solution was heated in a water bath to 70-80°C for 1 hour. In another container was added 1 g of itraconazole (Wyckoff). Three mL of NMP were added and the mixture heated to 70-80°C to effect dissolution (approximately 30 minutes). Phospholipid (Lipoid S-100) was added to this hot solution.' Heating was continued at 70-90°C for 30 minutes until all of the phospholipid was dissolved. The hydroxyethylstarch solution was combined with the itraconazole/ phospholipid solution. This mixture was heated for another 30 minutes at 80-95°C to dissolve the mixture.

Addition of Solution A to Tris Buffer: Ninety-four (94) mL of 50 mM tris(hydroxymethyl)aminomethane buffer was cooled in an ice bath. As the tris solution was being rapidly stirred, the hot Solution A (see above) was slowly added dropwise (less than 2 cc/minute).

After complete addition, the resulting suspension was sonicated (Cole-Parmer Ultrasonic Processor - 20,000 Hz, 80% amplitude setting) while still being cooled in the ice bath. A one-inch solid probe was utilized. Sonication was continued for 5 minutes. The ice bath was removed, the probe was removed and retuned, and the probe was again immersed in the suspension. The suspension was sonicated again for another 5 minutes without the ice bath. The sonicator probe was once again removed and retuned, and after immersion of the probe the sample was sonicated for another 5 minutes. At this point, the temperature of the suspension had risen to 82°C. The suspension was quickly cooled again in an ice bath and when it was found to be below room temperature it was poured into a Type I glass bottle and sealed. Microscopic visualization of the particles indicated individual particle sizes on the order of one micron or less.

After one year of storage at room temperature, the suspension was reevaluated for particle size and found to have a mean diameter of approximately 300 nm.

Example 15: Prophetic example of Method A using HES

The present invention contemplates preparing a 1% itraconazole nanosuspension with hydroxyethylstarch utilizing Method A by following the steps of Example 14 with the exception the HES would be added to the tris buffer solution instead of to the NMP solution. The aqueous solution may have to be heated to dissolve the HES.

Example 16: Seeding during Homogenization to Convert a Mixture of Polymorphs to the More Stable Polymorph

Sample preparation. An itraconazole nanosuspension was prepared by a microprecipitation-homogenization method as follows. Itraconazole (3g) and Solutol HR (2.25g) were dissolved in 36mL of N-methyl-2-pyrrolidinone (NMP) with low heat and stirring to form a drug concentrate solution. The solution was cooled to room temperature and filtered through a 0.2 μm nylon filter under vacuum to remove undissolved drug or particulate matter. The solution was viewed under polarized light to ensure that no crystalline material was present after filtering. The drug concentrate solution was then added at 1.0 mL/minute to approximately 264 mL of an aqueous buffer solution (22 g/L glycerol in 5 mM tris buffer). The aqueous solution was kept at 2-3°C and was continuously stirred at approximately 400 rpm during the drug concentrate addition. Approximately 100 mL of the resulting suspension was centrifuged and the solids re-suspended in a pre-filtered solution of 20% NMP in water. This suspension was re-centrifuged and the solids were transferred to a vacuum oven for overnight drying at 25°C. The resulting solid sample was labeled SMP 2 PRE.

<u>Sample characterization</u>. The sample SMP 2 PRE and a sample of the raw material itraconazole were analyzed using powder x-ray diffractometry. The measurements were performed using a Rigaku MiniFlex+ instrument with copper radiation, a step size of 0.02° 2θ and scan speed of 0.25° 2θ/minute. The resulting powder diffraction patterns are shown in Figure 12. The patterns show that SMP-2-PRE is significantly different from the raw material, suggesting the presence of a different polymorph or a pseudopolymorph.

Differential scanning calorimetry (DSC) traces for the samples are shown in Figures 13a and 13b. Both samples were heated at 2°/min to 180°C in hermetically sealed aluminum pans.

The trace for the raw material itraconazole (Figure 13a) shows a sharp endotherm at approximately 165°C.

The trace for SMP 2 PRE (Figure 13b) exhibits two endotherms at approximately 159°C and 153°C. This result, in combination with the powder x-ray diffraction patterns, suggests that SMP 2 PRE consists of a mixture of polymorphs, and that the predominant form is a polymorph that is less stable than polymorph present in the raw material.

Further evidence for this conclusion is provided by the DSC trace in Figure 14, which shows that upon heating SMP 2 PRE through the first transition, then cooling and reheating, the less stable polymorph melts and recrystallizes to form the more stable polymorph.

<u>Seeding</u>. A suspension was prepared by combining 0.2g of the solid SMP 2 PRE and 0.2g of raw material itraconazole with distilled water to a final volume of 20 mL (seeded sample). The suspension was stirred until all the solids were wetted. A second suspension was prepared in the same manner but without adding the raw material itraconazole (unseeded sample). Both suspensions were homogenized at approximately 18,000 psi for 30 minutes. Final temperature of the suspensions after homogenization was approximately 30°C. The suspensions were then centrifuged and the solids dried for approximately 16 hours at 30°C.

Figure 15 shows the DSC traces of the seeded and unseeded samples. The heating rate for both samples was 2°/min to 180°C in hermetically sealed aluminum pans. The trace for the unseeded sample shows two endotherms, indicating that a mixture of polymorphs is still present after homogenization. The trace for the seeded sample shows that seeding and homogenization causes the conversion of the solids to the stable polymorph. Therefore, seeding appears to influence the kinetics of the transition from the less stable to the more stable polymorphic form.

Example 17: Seeding during Precipitation to Preferentially Form a Stable Polymorph

<u>Sample preparation</u>. An itraconazole-NMP drug concentrate was prepared by dissolving 1.67g of itraconazole in 10mL of NMP with stirring and gentle heating. The solution was filtered twice using 0.2 μm syringe filters. Itraconazole nanosuspensions were then prepared by adding 1.2 mL of the drug concentrate to 20 mL of an aqueous receiving solution at approx. 3°C and stirring at approx. 500 rpm. A seeded nanosuspension was prepared by using a mixture of approx. 0.02g of raw material itraconazole in distilled water as the receiving solution. An unseeded nanosuspension was prepared by using distilled water only as the receiving solution. Both suspensions were centrifuged, the supernatants decanted, and the solids dried in a vacuum oven at 30°C for approximately 16 hours.

<u>Sample characterization</u>. Figure 16 shows a comparison of the DSC traces for the solids from the seeded and unseeded suspensions. The samples were heated at 2°/min to 180°C in hermetically sealed aluminum pans. The dashed line represents the unseeded sample, which shows two endotherms, indicating the presence of a polymorphic mixture.

The solid line represents the seeded sample, which shows only one endotherm near the expected melting temperature of the raw material, indicating that the seed material induced the exclusive formation of the more stable polymorph.

Example 18: Polymorph control by seeding the drug concentrate

<u>Sample preparation</u>. The solubility of itraconazole in NMP at room temperature (approximately 22 °C) was experimentally determined to be 0.16 g/mL. A 0.20 g/mL drug concentrate solution was prepared by dissolving 2.0 g of itraconazole and 0.2 g Poloxamer 188 in 10 mL NMP with heat and stirring. This solution was then allowed to cool to room temperature to yield a supersaturated solution. A microprecipitation experiment was immediately performed in which 1.5 mL of the drug concentrate was added to 30 mL of an aqueous solution containing 0.1% deoxycholate, 2.2% glycerol. The aqueous solution was maintained at ~2°C and a stir rate of 350 rpm during the addition step. The resulting presuspension was homogenized at ~13,000 psi for approx. 10 minutes at 50 °C. The suspension was then centrifuged, the supernatant decanted, and the solid crystals dried in a vacuum oven at 30 °C for 135 hours.

The supersaturated drug concentrate was subsequently aged by storing at room temperature in order to induce crystallization. After 12 days, the drug concentrate was hazy, indicating that crystal formation had occurred. An itraconazole suspension was prepared from the drug concentrate, in the same manner as in the first experiment, by adding 1.5 mL to 30 mL of an aqueous solution containing 0.1% deoxycholate, 2.2% glycerol. The aqueous solution was maintained at ~5 °C and a stir rate of 350 rpm during the addition step. The resulting presuspension was homogenized at ~13,000 psi for approx. 10 minutes at 50°C. The suspension was then centrifuged, the supernatant decanted, and the solid crystals dried in a vacuum oven at 30 °C for 135 hours.

Sample characterization. X-ray powder diffraction analysis was used to determine the morphology of the dried crystals. The resulting patterns are shown in Figure 17. The crystals from the first experiment (using fresh drug concentrate) were determined to consist of the more stable polymorph. In contrast, the crystals from the second experiment (aged drug concentrate) were predominantly composed of the less stable polymorph, with a small amount of the more stable polymorph also present. Therefore, it is believed that aging induced the formation of crystals of the less stable polymorph in the drug concentrate, which then acted as seed material during the microprecipitation and homogenization steps such that the less stable polymorph was preferentially formed.

Figure 12: X-ray powder diffraction patterns for raw material itraconazole (top) and SMP-2-PRE (bottom). The raw material pattern has been shifted upward for clarity. (Example 16)

Figure 13a: DSC trace for raw material itraconazole (Example 16)

Figure 13b: DSC trace for SMP-2-PRE. (Example 16)

Figure 14: DSC trace for SMP-2-PRE showing the melt of the less stable polymorph upon heating to 160 °C, a recrystallization event upon cooling, and the subsequent melting of the more stable polymorph upon reheating to 180 °C. (Example 16)

Figure 15: Comparison of SMP-2-PRE samples after homogenization. Solid line = sample seeded with raw material itraconazole. Dashed line = unseeded sample. The solid line has been shifted by 1 W/g for clarity (Example 16)

Figure 16: Effect of seeding during precipitation. Dashed line = unseeded sample, solid line = sample seeded with raw material itraconazole. The unseeded trace (dashed line) has been shifted upward by 1.5 W/g for clarity. (Example 17)

Figure 17: Effect of seeding the drug concentrate through aging. Top x-ray diffraction pattern is for crystals prepared from fresh drug concentrate, and is consistent with the stable polymorph (see Figure 12, top). Bottom pattern is for crystals prepared from aged (seeded) drug concentrate, and is consistent with the metastable polymorph (see Figure 12, bottom). The top pattern has been shifted upward for clarity. (Example 18)

## Claims

1. A method for preparing submicron sized particles of a pharmaceutically-active compound, the solubility of which is greater in a water-miscible first solvent than in a second solvent which is aqueous, the process comprising the steps of:

(i) dissolving the pharmaceutically-active compound in the water-miscible first solvent to form a solution, the first solvent being selected from the group consisting of N-methyl-2-pyrrolidinone, 2-pyrrolidone, dimethyl sulfoxide, dimethylacetamide, lactic acid, methanol, ethanol, isopropanol, 3-pentanol, n-propanol, glycerol butylene glycol, ethylene glycol, propylene glycol, mono- and diacylated monoglycerides, dimethyl isosorbide, acetone, dimethylformamide, 1,4-dioxane, ethyl acetate, propyl acetate, polyethylene glycol, polyethylene glycol esters, polyethylene glycol sorbitans, polyethylene glycol monoalkyl ethers, polypropylene glycol, polypropylene alginate, propylene glyr-ol-10 butanediol, propylene glycol-10 methyl glucose ether, propylene glycol-20 methyl glucose ether, propylene glycol-15 stearyl ether, propylene glycol dicaprylate, propylene glycol dicaprate, propylene glycol laurate;

(ii) mixing the solution with the second solvent to define a pre-suspension; and

(iii) adding energy to the pre-suspension to form particles having an average effective particle size of less than about 2 μm, and said adding energy step comprises homogenization, counter-current flow homogenization, microfluidization or sonication.

2. The method of claim 1 wherein the pre-suspension produced in step (ii) comprises said pharmaceutically-active compound in an amorphous form, in a semicrystalline form or in a supercooled liquid form and having a given average effective particle size; and in step (iii) the pre-suspension is annealed to form pharmaceutically-active compound particles having essentially the same average effective particle size of the pre-suspension and in a more stable form.

3. The method of claim 1 wherein said mixing of the solution with the second solvent forms the pre-suspension with particles in a needle-like friable form.

4. The method of any of claims 1, 2 or 3 further comprising the step of mixing into the second solvent one or more surface modifiers selected from the group consisting of anionic surfactants, cationic surfactants, nonionic surfactants and surface active biological modifiers, optionally in two or three steps.

5. The method of any of claims 1, 2 or 3 further comprising the step of mixing into the solution one or more surface modifiers selected from the group consisting of anionic surfactants, cationic surfactants, nonionic surfactants and surface active biological modifiers, optionally in any of two to five steps.

6. The method of claim 4 or 5 wherein the nonionic surfactant is selected from the group consisting of polyoxyethylene fatty alcohol ethers, polyoxyethylene sorbitan fatty acid esters, polyoxyethylene fatty acid esters, sorbitan esters, glycerol monostearate, polyethylene glycols, polypropylene glycols, cetyl alcohol, cetostearyl alcohol, stearyl alcohol, aryl alkyl polyether alcohols, polyoxyemylene-polyoxypropylene copolymers, polaxamines, methylcellulose, hydroxycellulose, hydroxy propylcellulose, hydroxy propylmethylcellulose, noncrystalline cellulose, polysaccharides, starch, starch derivatives, hydroxyethylstarch, polyvinyl alcohol, and polyvinylpyrrolidone.

7. The method of claim 4 or 5 wherein the anionic surfactant is selected from the group consisting of potassium laurate, ttriethanolamine stearate, sodium lauryl sulfate, sodium dodecylsulfate, alkyl polyoxyethylene sulfates, sodium alginate, dioctyl sodium sulfosuccinate, phosphatidyl glycerol, phosphatidyl inositol, phosphatidylserine, phosphatidic acid and their salts, glyceryl esters, sodium carboxymethylcellulose, bile acids and their salts, cholic acid, deoxycholic acid, glycocholic acid, taurocholic acid, glycodeoxycholic acid, and calcium carboxymethylcellulose.

8. The method of claim 4 or 5 wherein the cationic surfactant is selected from the group consisting of quaternary ammonium compounds, benzalkonium chloride, cetyltrimethylammonium bromide, chitosans and lauryldimethylbenzylammonium chloride.

9. The method of claim 4 or 5 wherein the surface active biological modifiers are selected from the group consisting of albumin, casein, heparin, hirudin, or other proteins.

10. The method of claim 4 wherein the first solvent is N-methyl-2-pyrrolidinone.

11. The method of claim 10 wherein the surface modifier is a copolymer of oxyethylene and oxypropylene.

12. The method of claim 11 wherein the copolymer of oxyethylene and oxypropylene is a block copolymer.

13. The method of claim 4 or 5 wherein at least one of the surface modifiers is a bile acid or a salt thereof.

**14.** The method of claim 4 or 5 wherein at least one of the second surface modifiers is selected from deoxycholic acid, glycocholic acid, glycodeoxycholic acid, taurocholic acid and salts of these acids.

**15.** The method of claim 4 further comprising the step of adding a pH adjusting agent to the second solvent.

**16.** The method of claim 15 wherein the pH adjusting agent is selected from the group consisting of sodium hydroxide, hydrochloric acid, tris buffer, citrate buffer, acetate, lactate, and meglumine.

**17.** The method of claim 15 wherein the pH adjusting agent is added to the second solvent to bring the pH of the second solvent within the range of from about 3 to about 11.

**18.** The method of claim 1 further comprising the step of mixing into the second solvent a phospholipid.

**19.** The method of claim 18 wherein the phospholipid is selected from natural phospholipids and synthetic phospholipids.

**20.** The method of claim 18 wherein the phospholipid is selected from the group consisting of phosphatidylcholine, phosphatidylethanolamine, phosphatidylserine, phosphatidylinositol, phosphatidylglycerol, phosphatidic acid, lysophospholipids, egg phospholipid and soybean phospholipid.

**21.** The method of claim 2 wherein the adding energy step includes the step of converting the particles of the pre-suspension to a crystalline form as determined by DSC.

**22.** The method of claim 2 wherein the particles after the adding energy step have a reduced tendency to aggregate into larger particles when compared to the particles of the pre-suspension.

**23.** The method of claim 2 wherein the particles of the pre-suspension have an average effective particle size of from about 2 $\mu$m to about 50 nm.

**24.** The method of claim 2 wherein the particles of the pre-suspension have an average effective particle size of less than about 400 nm.

**25.** The method of any of claims 1 to 24 wherein said adding energy step comprises homogenization, counter-current flow homogenization or microfluidization.

## Patentansprüche

**1.** Verfahren zur Herstellung von Teilchen von Submikrometergröße einer pharmazeutisch aktiven Verbindung, dessen Löslichkeit größer in einem mit Wasser mischbaren ersten Lösungsmittel als in einem zweiten Lösungsmittel ist, welches wäßrig ist, wobei das Verfahren die Schritte umfaßt:

(i) Lösen der pharmazeutisch aktiven Verbindung in dem mit Wasser mischbaren ersten Lösungsmittel unter Bildung einer Lösung, wobei das erste Lösungsmittel aus der Gruppe, bestehend aus N-Methyl-2-pyrrolidinon, 2-Pyrrolidon, Dimethylsulfoxid, Dimethylacetamid, Milchsäure, Methanol, Ethanol, Isopropanol, 3-Pentanol, n-Propanol, Glycerin, Butylenglykol, Ethylenglykol, Propylenglykol, mono- und diacylierten Monoglyceriden, Dimethylisosorbid, Aceton, Dimethylformamid, 1,4-Dioxan, Ethylacetat, Propylacetat, Polyethylenglykol, Polyethylenglykolestem, Polyethylenglykolsorbitanen, Polyethylenglykolmonoalkylethern, Polypropylenglykol, Polypropylenalginat, Propylenglykol-10-butandiol, Propylenglykol-10-methylglukoseether, Propylenglykol-20-methylglukoseether, Propylenglykol-15-stearylether, Propylenglykoldicaprylat, Propylenglykoldicaprat, Propylenglykollaurat, ausgewählt ist,
(ii) Mischen der Lösung mit dem zweiten Lösungsmittel unter Definieren einer Vorsuspension, und
(iii) Zugeben von Energie zu der Vorsuspension unter Bildung von Teilchen mit einer durchschnittlichen wirksamen Teilchengröße von weniger als etwa 2 $\mu$m, und wobei der Schritt des Zugebens von Energie Homogenisierung, Homogenisieren im Gegenstromfluß, Mikrofluidisation und Ultraschall umfaßt.

**2.** Verfahren gemäß Anspruch 1, wobei die in Schritt (ii) erzeugte Vorsuspension die pharmazeutisch aktive Verbindung in einer amorphen Form, in einer semikristallinen Form oder in einer unterkühlten flüssigen Form umfaßt und eine gegebene durchschnittliche wirksame Teilchengröße aufweist, und in Schritt (iii) die Vorsuspension unter Bildung von Teilchen der pharmazeutisch aktiven Verbindung mit im wesentlichen der gleichen durchschnittlichen wirksamen Teilchengröße der Vorsuspension und in einer stabileren Form getempert wird.

3. Verfahren gemäß Anspruch 1, wobei das Mischen der Lösung mit dem zweiten Lösungsmittel die Vorsuspension mit Teilchen in einer nadelförmigen zerreibbaren Form bildet.

4. Verfahren gemäß einem der Ansprüche 1, 2 oder 3, weiter umfassend den Schritt des Mischens von einem oder mehreren grenzflächenaktiven Modifizierungsmitteln, ausgewählt aus der Gruppe, bestehend aus anionischen grenzflächenaktiven Mitteln, kationischen grenzflächenaktiven Mitteln, nichtionischen grenzflächenaktiven Mitteln und grenzflächenaktiven biologischen Modifizierungsmitteln, in das zweite Lösungsmittel, gegebenenfalls in zwei oder drei Schritten.

5. Verfahren gemäß einem der Ansprüche 1, 2 oder 3, weiter umfassend den Schritt des Mischens von einem oder mehreren grenzflächenaktiven Modifizierungsmitteln, ausgewählt aus der Gruppe, bestehend aus anionischen grenzflächenaktiven Mitteln, kationischen grenzflächenaktiven Mitteln, nichtionischen grenzflächenaktiven Mitteln und grenzflächenaktiven biologischen Modifizierungsmitteln in die Lösung, gegebenenfalls in jedwedem von zwei bis fünf Schritten.

6. Verfahren gemäß Anspruch 4 oder 5, wobei das nicht-ionische grenzflächenaktive Mittel aus der Gruppe, bestehend aus Polyoxyethylenfettalkoholethern, Polyoxyethylensorbitfettsäureestern, Polyoxyethylenfettsäureestern, Sorbitestern, Glycerinmonostearat, Polyethylenglykolen, Polypropylenglykolen, Cetylalkohol, Cetostearylalkohol, Stearylalkohol, Arylalkylpolyetheralkoholen, Polyoxyethylen-Polyoxypropylen-Copolymeren, Polaxaminen, Methylcellulose, Hydroxycellulose, Hydroxypropylcellutose, Hydroxypropytmethylcellulose, nicht-kristalliner Cellulose, Polysacchariden, Stärke, Stärkederivaten, Hydroxyethylstärke, Polyvinylalkohol und Polyvinylpyrrolidon, ausgewählt ist.

7. Verfahren gemäß Anspruch 4 oder 5, wobei das anionische grenzflächenaktive Mittel aus der Gruppe, bestehend aus Kaliumlaurat, Triethanolaminstearat, Natriumlaurylsulfat, Natriumdodecylsulfat, Alkylpolyoxyethylensulfaten, Natriumalginat, Dioctylnatriumsulfosuccinat, Phosphatidylglycerin, Phosphatidylinositol, Phosphatidylserin, Phosphatidsäure und deren Salzen, Glycerylestem, Natriumcarboxymethylcellulose, Gallensäuren und deren Salzen, Cholsäure, Deoxycholsäure, Glycocholsäure, Taurocholsäure, Glycodeoxycholsäure und Calciumcarboxymethylcellulose, ausgewählt ist.

8. Verfahren gemäß Anspruch 4 oder 5, wobei das kationische grenzflächenaktive Mittel aus der Gruppe, bestehend aus quaternären Ammoniumverbindungen, Benzalkoniumchlorid, Cetyltrimethylammoniumbromid, Chitosanen und Lauryldimethylbenzylammoniumchlorid, ausgewählt ist.

9. Verfahren gemäß anspruch 4 oder 5, wobei die grenzflächenaktiven biologischen Modifizierungsmittel aus der Gruppe, bestehend aus Albumin, Kasein, Heparin, Hirudin oder anderen Proteinen, ausgewählt sind.

10. Verfahren gemäß Anspruch 4, wobei das erste Lösungsmittel N-Methyl-2-pyrrolidinon ist.

11. Verfahren gemäß Anspruch 10, wobei das grenzflächenaktive Modifizierungsmittel ein Copolymer von Oxyethylen und Oxypropylen ist.

12. Verfahren gemäß Anspruch 11, wobei das Copolymer von Oxyethylen und Oxypropylen ein Blockcopolymer ist.

13. Verfahren gemäß Anspruch 4 oder 5, wobei mindestens eines der grenzflächenaktiven Modifizierungsmittel eine Gallensäure oder ein Salz davon ist.

14. Verfahren gemäß Anspruch 4 oder 5, wobei mindestens eines der zweiten grenzflächenaktiven Modifizierungsmittel aus Deoxycholsäure, Glycocholsäure, Glycodeoxycholsäure, Taurocholsäure und Salzen dieser Säuren, ausgewählt ist.

15. Verfahren gemäß Anspruch 4, weiter umfassend den Schritt des Zugebens eines pH-Regulierungsmittels zu dem zweiten Lösungsmittel.

16. Verfahren gemäß Anspruch 15, wobei das pH-Regulierungsmittel aus der Gruppe, bestehend aus Natriumhydroxid, Salzsäure, Trispuffer, Citratpuffer, Acetat, Lactat und Meglumin, ausgewählt ist.

**17.** Verfahren gemäß Anspruch 15, wobei das pH-Regulierungsmittel zu dem zweiten Lösungsmittel zugegeben wird, um den pH des zweiten Lösungsmittels innerhalb des Bereichs von etwa 3 bis etwa 11 zu bringen.

**18.** Verfahren gemäß Anspruch 1, weiter umfassend den Schritt des Mischens eines Phospholipids in das zweite Lösungsmittel.

**19.** Verfahren gemäß Anspruch 18, wobei das Phospholipid aus natürlichen Phospholipiden und synthetischen Phospholipiden ausgewählt ist.

**20.** Verfahren gemäß Anspruch 18, wobei das Phospholipid aus der Gruppe, bestehend aus Phosphatidylcholin, Phosphatidylethanolamin, Phosphatidylserin, Phosphatidylinositol, Phosphatidylglycerin, Phosphatidsäure, Lysophospholipiden, Eiphospholipid und Sojabohnenphospholipid, ausgewählt ist.

**21.** Verfahren gemäß Anspruch 2, wobei der Schritt des Zugebens von Energie den Schritt des Umwandelns der Teilchen der Vorsuspension zu einer kristallinen Form, wie durch DSC bestimmt, einschließt.

**22.** Verfahren gemäß Anspruch 2, wobei die Teilchen nach dem Schritt des Zugebens von Energie eine verminderte Tendenz haben, in größere Teilchen zu aggregieren, wenn verglichen zu den Teilchen der Vorsuspension.

**23.** Verfahren gemäß Anspruch 2, wobei die Teilchen der Vorsuspension eine durchschnittliche wirksame Teilchengröße von etwa 2 $\mu$m bis etwa 50 nm aufweisen.

**24.** Verfahren gemäß Anspruch 2, wobei die Teilchen der Vorsuspension eine durchschnittliche wirksame Teilchengröße von weniger als etwa 400 nm aufweisen.

**25.** Verfahren gemäß einem der Ansprüche 1 bis 24, wobei der Schritt des Zugebens von Energie Homogenisierung, Homogenisierung im Gegenstromfluß oder Mikrofluidisation umfaßt.

**Revendications**

**1.** Procédé de préparation de particules submicroniques d'un composé actif d'un point de vue pharmaceutique, dont la solubilité est plus élevée dans un premier solvant miscible à l'eau que dans un second solvant qui est aqueux, le procédé comprenant les étapes consistant à :

(i) dissoudre le composé actif d'un point de vue pharmaceutique dans le premier solvant miscible à l'eau pour former une solution, le premier solvant étant choisi dans le groupe constitué par la N-méthyl-2-pyrrolidinone, la 2-pyrrolidone, le diméthyl sulfoxyde, le diméthylacétamide, l'acide lactique, le méthanol, l'éthanol, l'isopropanol, le 3-pentanol, le n-propanol, le glycérol, le butylène glycol, l'éthylène glycol, le propylène glycol, les monoglycérides mono et diacylés, l'isosorbide de diméthyle, l'acétone, le diméthylformamide, le 1,4-dioxanne, l'acétate d'éthyle, l'acétate de propyle, le polyéthylène glycol, les esters de polyéthylène glycol, les polyéthylène glycol sorbitanes, les éthers de monoalkyle et de polyéthylène glycol, le polypropylène glycol, l'alginate de polypropylène, le propylène glycol-10 butanediol, le propylène glycol-10 méthyl glucose éther, le propylène glycol-20 méthyl glucose éther, le propylène glycol-15 stéaryl éther, le dicaprylate de propylène glycol, le dicaprate de propylène glycol, le laurate de propylène glycol;
(ii) mélanger la solution à un second solvant pour définir une pré-suspension ; et
(iii) ajouter de l'énergie à la pré-suspension pour former des particules ayant une taille effective moyenne de particule inférieure à environ 2 $\mu$m, et ladite étape consistant à ajouter de l'énergie comprend l'homogénéisation, l'homogénéisation à contre-courant, la microfluidisation ou la sonication.

**2.** Procédé selon la revendication 1 dans lequel la pré-suspension produite dans l'étape (ii) comprend ledit composé actif d'un point de vue pharmaceutique sous une forme amorphe, sous une forme semi-cristalline ou sous une forme liquide en surfusion et ayant une taille effective moyenne de particule donnée ; et dans l'étape (iii), la pré-suspension est chauffée et refroidie lentement pour former des particules du composé actif d'un point de vue pharmaceutique ayant essentiellement la même taille effective moyenne de particule que la pré-suspension et sous une forme plus stable.

**3.** Procédé selon la revendication 1 dans lequel ledit mélange de la solution au second solvant forme la pré-suspension dans laquelle les particules ont la forme d'aiguilles friables.

4. Procédé selon l'une quelconque des revendications 1, 2 ou 3 comprenant en outre l'étape consistant à mélanger dans le second solvant un ou plusieurs tensioactifs choisis dans le groupe constitué par des tensioactifs anioniques, des tensioactifs cationiques, des tensioactifs non-ioniques et des tensioactifs biologiques, facultativement en deux ou trois étapes.

5. Procédé selon l'une quelconque des revendications 1, 2 ou 3 comprenant en outre l'étape consistant à mélanger dans la solution un ou plusieurs tensioactifs choisis dans le groupe constitué par des tensioactifs anioniques, des tensioactifs cationiques, des tensioactifs non-ioniques et des tensioactifs biologiques, facultativement en deux, trois, quatre ou cinq étapes.

6. Procédé selon la revendication 4 ou 5 dans lequel le tensioactif non-ionique est choisi dans le groupe constitué par les éthers d'alcool gras et de polyoxyéthylène, les esters de polyoxyéthylène sorbitane d'acide gras, les esters de polyoxyéthylène d'acide gras, les esters de sorbitane, le monostéarate de glycérol, les polyéthylène glycols, les polypropylène glycols, l'alcool cétylique, l'alcool cétostéarylique, l'alcool stéarylique, les aryl alkyl polyéther alcools, les copolymères polyoxyéthylène-polyoxypropylène, les polaxamines, la méthylcellulose, l'hydroxycellulose, l'hydroxy propylcellulose, l'hydroxy propylméthylcellulose, la cellulose non cristalline, les polysaccharides, l'amidon, les dérivés de l'amidon, l'hydroxyéthyl amidon, l'alcool polyvinylique, et la polyvinylpyrrolidone.

7. Procédé selon la revendication 4 ou 5 dans lequel le tensioactif anionique est choisi dans le groupe constitué par le laurate de potassium, le stéarate de triéthanolamine, le sulfate de lauryle et de sodium, le dodécylsulfate de sodium, les polyoxyéthylène sulfates d'alkyle, l'alginate de sodium, le sulfosuccinate de dioctyle et de sodium, le phosphatidyl glycérol, le phosphatidyl inositol, la phosphatidylsérine, l'acide phosphatidique et leurs sels, les esters de glycéryle, la carboxyméthylcellulose de sodium, les acides biliaires et leurs sels, l'acide cholique, l'acide déoxycholique, l'acide glycocholique, l'acide taurocholique, l'acide glycodéoxycholique, et la carboxyméthylcellulose de calcium.

8. Procédé selon la revendication 4 ou 5 dans lequel le tensioactif cationique est choisi dans le groupe constitué par les composés d'ammonium quaternaire, le chlorure de benzalkonium, le bromure de cétyltriméthylammonium, les chitosanes et le chlorure de lauryldiméthylbenzylammonium.

9. Procédé selon la revendication 4 ou 5 dans lequel les tensioactifs biologiques sont choisis dans le groupe constitué par l'albumine, la caséine, l'héparine, l'hirudine ou d'autres protéines.

10. Procédé selon la revendication 4 dans lequel le premier solvant est la N-méthyl-2-pyrrolidinone.

11. Procédé selon la revendication 10 dans lequel le tensioactif est un copolymère de l'oxyéthylène et de l'oxypropylène.

12. Procédé selon la revendication 11 dans lequel le copolymère de l'oxyéthylène et de l'oxypropylène est un copolymère séquencé.

13. Procédé selon la revendication 4 ou 5 dans lequel au moins un des tensioactifs est un acide biliaire ou son sel.

14. Procédé selon la revendication 4 ou 5 dans lequel au moins un des seconds tensioactifs est choisi parmi l'acide déoxycholique, l'acide glycocholique, l'acide glycodéoxycholique, l'acide taurocholique et les sels de ces acides.

15. Procédé selon la revendication 4 comprenant en outre l'étape consistant à ajouter un agent ajustant le pH au second solvant.

16. Procédé selon la revendication 15 dans lequel l'agent ajustant le pH est choisi dans le groupe constitué par l'hydroxyde de sodium, l'acide chlorhydrique, le tampon tris, le tampon citrate, l'acétate, le lactate et la méglumine.

17. Procédé selon la revendication 15 dans lequel l'agent ajustant le pH est ajouté au second solvant pour amener le pH du second solvant entre environ 3 et environ 11.

18. Procédé selon la revendication 1 comprenant en outre l'étape consistant à mélanger dans le second solvant un phospholipide.

**19.** Procédé selon la revendication 18 dans lequel le phospholipide est choisi parmi les phospholipides naturels et les phospholipides synthétiques.

**20.** Procédé selon la revendication 18 dans lequel le phospholipide est choisi dans le groupe constitué par la phosphatidylcholine, la phosphatidyléthanolamine, la phosphatidylsérine, le phosphatidylinositol, le phosphatidylglycérol, l'acide phosphatidique, les lysophospholipides, le phospholipide d'oeuf et le phospholipide de soja.

**21.** Procédé selon la revendication 2 dans lequel l'étape consistant à ajouter de l'énergie comprend l'étape consistant à convertir les particules de la pré-suspension en une forme cristalline telle que déterminée par DSC.

**22.** Procédé selon la revendication 2 dans lequel les particules après l'étape consistant à ajouter de l'énergie ont moins tendance à s'agréger en particules plus grosses par rapport aux particules de la pré-suspension.

**23.** Procédé selon la revendication 2 dans lequel les particules de la pré-suspension possèdent une taille effective moyenne de particule d'environ 2 $\mu$m à environ 50 nm.

**24.** Procédé selon la revendication 2 dans lequel les particules de la pré-suspension possèdent une taille effective moyenne de particule inférieure à environ 400 nm.

**25.** Procédé selon les revendications 1 à 24 dans lequel ladite étape consistant à ajouter de l'énergie comprend l'homogénéisation, l'homogénéisation à contre-courant ou la microfluidisation.